# EUROPEAN PATENT APPLICATION

(11) **EP 1 927 365 A1**
(43) Date of publication of application: **04.06.2008**
(21) Application number: 06741788.1
(22) Date of filing: 30.04.2006
(51) Int. Cl.: A61K 38/48, A61P 9/00, C12N 1/20

(54) **A NEW BACILLUS SUBTILIS STRAIN AND ITS USE IN PREPARING MEDICINE FOR TREATING THROMBOSIS**

(30) Priority: 30.04.2005 CN 200510020836
(71) Applicant: Chengdu Di' Ao Jiuhong Pharmaceutical Factory, High Tech Development Zone Chengdu, Sichuan 610041 (CN)
(72) Inventor: CHEN, Jun, Chengdu, Sichuan 610041 (CN); WEI, Qi, Chengdu, Sichuan 610041 (CN); JIANG, Fei, Chengdu, Sichuan 610041 (CN); LIU, Zhongrong, Chengdu, Sichuan 610041 (CN); LAI, Yana, Chengdu, Sichuan 610041 (CN); HE, Junrong, Chengdu, Sichuan 610041 (CN); LI, Bogang, Chengdu, Sichuan 610041 (CN)
(74) Representative: Le Coupanec, Pascale A.M.P.
(86) International application number: PCT/CN2006/000892
(87) International publication number: WO 2006/116949

(57) **Abstract**

A new *Bacillus subtilis* strain and its use in preparing medicine for treating thrombosis are provided. Particularly, the use of fibrinolytic enzyme produced by *Bacillus subtilis* in preparing medicine for treating thrombosis is provided. The present invention also discloses a method for producing said fibrinolytic enzyme (nattokinase). It is efficient and cost-effective to prepare nattokinase by using the new *Bacillus subtilis* strain of the invention, suitable for mass industrial production.

## Description

### TECHNICAL FIELD

The present invention relates to a new strain of *Bacillus subtilis* and its use in preparing pharmaceuticals for treating thrombosis, the use of the fibinolysin produced by *Bacillus subtilis* in preparing drugs for treating thrombosis in particular. In general, this invention belongs to the field of biological medicine.

### BACKGROUND OF THE INVENTION

Thrombosis is one of the principal diseases endangering human health. For example, the death rates of cerebral thrombosis and acute myocardial infarction have reached above 40%, making thrombosis a disease of the highest death rate. Thrombolysis is an important method for the treatment of this kind of diseases. However the thrombolytic drugs that are presently used to treat thrombosis in clinical practice, such as Streptokinase (SK), Urokinase (UK) and Recombinant Tissue-type Plasminogen Activator (rt-PA), all have certain defects including strong toxicity, serious side effects, short time of half life in vivo and expensive prices, etc.

Nattokinase (NK) is a kind of serine protease extracted from a traditional Japanese food called Natto. This plasmin consists of 275 amino acids and has no toxicity or side effect. Its relative molecular weight is 27728, far smaller than that of urokinase. Therefore, it is easy to be absorbed by the body as a drug- Its value of isoelectric focusing electrophoresis is 8.6±0.3 and it remains stable under the condition of pH 6.0 to 12.0 but will become unstable when the pH value is lower than 5.0. Human body experiments indicate that nattokinase not only has the direct effect of thrombolysis, but can also dissolve thrombus by activating plasminogen and pro-urokinase into fibrinolysin and urokinase respectively. At the same time, we have also found that nattokinase can activate the production of tissue-type plasminogen activator (t-PA) in blood plasma, presenting an indirect activity of thrombolysis. It is approved by test that NK has fast and sustaining curative effects.

The oral administration and fibrinolyic activity tests of the preparation of nattokinase showed that peak value of activity was reached 4 hours after taking the medicine. Then it began to decline and maintained certain activity till 8 hours later. Meanwhile, it was revealed that the quantity of internal tissue-type plasminogen activator (t-PA) also increased, indicating that besides the fibrinolytic activity of its own, nattokinase also increases the endogenic fibrinolytic activity by activating vascular endothelial cells to produce t-PA after being absorbed into blood through intestinal tract. Furthermore, nattokinase directly destroys cross-linked fibrin instead of fibrinogen in human body and the activity of its fibrinogen hydrolysis is far below that of plasmin and urokinase and remains the same as that of t-PA on the whole, indicating that natokinase is resistant to bleeding when it exhibits fibrinolyic activity.

Existing research work has shown that the bacterium producing nattokinase isolated from natto is mostly *Bacillus subtilis.* For example, the Chinese patent (CN 02116667.6) disclosed a strain of *Bacillus subtilis* producing nattokinase had the highest enzyme activity of 200 UK unit per milligram under the condition of liquid fermentation and 20 UK unit per milligram under the condition of solid fermentation respectively. Another Chinese patent (CN 00107589.6) also revealed a strain of *Bacillus subtilis* producing nattokinase. A few literatures also reported the optimization of conditions of screening and fermentation of *Bacillus subtilis natto* such as Mei Lehe *et al* who reported a strain of *Bacillus subtilis* had the activity of nattokinase of 970 IU/ml under shaking bed condition, and with the optimized condition in fermenters, the activity increased to 1314 IU/ml (Mei Lehe, Hu Sheng, Xu Jing, et al. Screening of Bacillus subtilis natto and optimization of nattokinase fermentation. Journal of Zhejiang University (Engineering Science). 2004, 38(10): 1355-1360.).

Xiong Xiaohui *el al* also reported that the yield of nattokinase of 2250 IU/ml in supernate in a 5 Litre fermentor was obtained using liquid fermentation by *Bacillus subtilis natto* at the optimized conditions (Xiong Xiaohui, Liang Jianguang, Xiong Qiang. Optimization of Nattokinase Production Using Liquid Fermentation. Food and Fermentation Industries. 2004, 30(1): 62-66.). Xie Qiuling *et al* also isolated one strain of bacteria from natto and the strain gave the highest enzyme activity of 787.1 urokinase units per milliliter at the 4^{th} day of fermentation (Xie Qiuling, Guo Yong. The optimization of fermentation conditions of nattokinase. Journal of South China University of Technology (Natural Science). 1999, 27(5): 127-131.). Bao Yianxia *et al* presented a method of the solid fermentation for producing nattokinase that made nattokinase activity reach 1577 U/g when the strain was grown in the medium consisting of bean dregs and weat bran and under such cultural conditions as initial moisture content of 65%, initial pH value of 8.0 and temperature of 35 degree C (Bao Yanxia, Chen Jun, Qian Zhiyu, et al. Optimization of the technique of solid fermentation of nattokinase. Journal of Shenyang Pharmaceutical University. 2004, 21 (6): 468-471.). Li Limin *et al* reported a method for nattokinase isolation and purification, i.e. by pretreatment with addition of CaCl₂ and Na₂C0₃, centrifugation, ultrafiltration, condensation and ion-exchange chromatography, the comparatively purified apozymase was obtained and gave a single band on SDS-PAGE (Li Limin, Hui Hongwen, Wang Mingfang. Study on purification of nattokinase from the B. natto fermented broth [liquor]. Journal of Inner Mongolia Agricultural University. 2003, 24(4): 51-54.).

Lu Ying *et al* described a method by which nattokinase extracted from natto was purified by ammionium sulfate fraction precipitation, hydrophobic interaction colum and ion exchange colum. The enzyme protein was shown to be a single homogeneous component with a molecular weight of 28 KD by SDS-PAGE (Lu Ying, Zhang Lu, Feng Lei, et al. Purification and Characterization of Nattokinase from Bacillus subtilis. Food and Fermentation Industries. 2004, 30(3): 122-124.). Bai Zhen *et al* indicated that nattokinase (NK) was isolated by (NH₄)₂SO₄ fractional precipitation, affinity chromatography, anion-exchange chromatography and gel filtration after extracted from natto with physiological saline and the purified NK gave a single sharp band on SDS-PAGE (Bai Zhen, Xu Mei, Han Siqin, et al. Purification and characterization of Nattokinase. Journal of Northeast Agricultural University. 2004, 35(6): 667-673.).

The yield of enzyme of *Bacillus subtilis* is generally not high with existing technology and so far there is no report on any effective oral drugs made thereof on market.

### SUMMARY OF THE INVENTION

One object of the present invention is to provide the application of a new strain of *Bacillus subtilis* in preparation of pharmaceuticals for treating thrombosis. Another object of the present invention is to provide a plasminokinase for treating thrombosis, specifically, the fibrinolysin produced by the new strain of *Bacillus subtilis.*

The invention also provides the use of the new strain of *Bacillus subtilis* in preparing pharmaceuticals for treating thrombosis and a fibrinolysin produced by the new strain of *Bacillus subtilis* (i.e. nattokinase) for the preparation of pharmaceuticals for treating thrombosis.

From commercially available natto, the inventors isolated a strain of Bacillus sp. 1507, the enzyme producing ability of which was 502 IU/ml and could be increased to 753 IU/ml with optimization of fermentation condition, but it still did not possess the value for industrial production. A novel strain of Bacillus sp. 1507 by mutation breeding was obtained and its appearance and physiological and biochemical characteristics are as follows:

| Test items | Results | Test items | Results |
|---|---|---|---|
| Gram staining | + | α-D-Lactose | +/- |
| Cell shape | rod shape | Maltobiose | + |
| >1.0 µm | + | D-Mannitol | - |
| Formation of endospore | + | D-Melizitose | - |
| Sporulation | subpolar | D-Melibiose | -/+ |
| Sporangiocyst intumescence | - | β-methyl-D-GI ucoside | - |
| Gemma spherical shape | oval shape | D-Allulose | - |
| Parasporal crystal | - | D-Melitriose | - |
| Catalase | + | Salicin | +/- |
| Oxidation enzyme | - | D-Sorbose | - |
| Amylohydroly sis | + | Sucrose | + |
| Gelatin liquefaction | + | D-Trehalose | + |
| D-Glucose | + | Xylitol | - |
| D-Xylose | - | Acetic acid | + |
| L-Arabinose | - | α-Hydroxybuty ric acid | - |
| D-Mannose | + | β-Hydroxybuty ric acid | - |
| Mannan | + | Pyruvic acid-methyl | + |
| Tween 80 | - | Succinate-met hyl | - |
| D- Arabitol | - | Propanoic acid | - |
| D-Cellobiose | - | Pyroracemic acid | + |
| D-Fructose | + | Amber acid | - |
| Fucose | - | L- Alanine | - |
| Citrate | + | Glycerol | + |
| Nitrate reduction | + | Hydrolysis caseinoge | + |
| D-Galactose | -/+ | Uridine | + |
| Gentiobiose | - | m-Inositol | - |

Although many kinds of fermentation techniques have been reported in existing literatures related to nattokinase from *Bacillus subtilis,* but the majority of the publicized strains have lower enzyme producing ability. On the other hand, other literatures describes some fermentation products from *Bacillus subtilis* that have higher enzyme producing ability, but it is difficult to evaluate the abilities of these publicized enzymes for the uncertainties of measuring conditions. The strain of *Bacillus subtilis* of the invention was fermented under the condition of temperature 30 to 40 degree C and with the speed of 180 to 220rpm for shaking bed, and the peak of enzyme production was obtained next day with enzyme activity of 1600 IU/ml and could be increased to 1697 IU/ml under optimized conditions. The enzyme activity of dried crude product could reach to 5000-10000 IU/g in solid fermentation of 10 kg supplies. It is believed that the enzyme activity production will be greatly enhanced after expansion of the scale of production. Therefore, it has a very high industrial application value. So we think that the strain of *Bacillus subtilis* (No. Diao 1502) is a newly developed, different one in comparison with any *Bacillus subtilis* producing nattokinase reported in existing literatures.

In the initial document, the strain of present invention was identified as *Bacillus cereus,* which was deposited in China Center for Type Culture Collection (CCTCC) (accession number of CCTCC M204092), according to its shape and characteristics of physiology and biochemistry. DNA sequencing was performed later at CCTCC and the result (16S rRNA serial) reads as follows:

CTTGTTACGACTTCACCCCAATCATCTGTCCCACCTTCGGCGGCTGGCTC
CTAAAAGGTTACCTCACCGACTTCGGGTGTTACAAACTCTCGTGGTGTGACG
GGCGGTGTGTACAAGGCCCGGGAACGTATTCACCGCGGCATGCTGATCCGCG
ATTACTAGCGATTCCAGCTTCACGCAGTCGAGTTGCAGACTGCGATCCGAAC
TGAGAACAGATTTGTGGGATTGGCTTAACCTCGCGGTTTCGCTGCCCTTTGTT
CTGTCCATTGTAGCACGTGTGTAGCCCAGGTCATAAGGGGCATGATGATTTGA
CGTCATCCCCACCTTCCTCCGGTTTGTCACCGGCAGTCACCTTAGAGTGCCCA
ACTGAATGCTGGCAACTAAGATCAAGGGTTGCGCTCGTTGCGGGACTTAACC
CAACATCTCACGACACGAGCTGACGACAACCATGCACCACCTGTCACTCTGC
CCCCGAAGGGGACGTCCTATCTCTAGGATTGTCAGAGGATGTCAAGACCTGG
TAAGGTTCTTCGCGTTGCTTCGAATTAAACCACATGCTCCACCGCTTGTGCGG
GCCCCCGTCAATTCCTTTGAGTTTCAGTCTTGCGACCGTACTCCCCAGGCGG
AGTGCTTAATGCGTTAGCTGCAGCACTAAGGGGCGGAAACCCCCTAACACTT
AGCACTCATCGTTTACGGCGTGGACTACCAGGGTATCTAATCCTGTTCGCTCC
CCACGCTTTCGCTCCTCAGCGTCAGTTACAGACCAGAGAGTCGCCTTCGCCA
CTGGTGTTCCTCCACATCTCTACGCATTTCACCGCTACACGTGGAATTCCACT
CTCCTCTTCTGCACTCAAGTTCCCCAGTTTCCAATGACCCTCCCCGGTTGAGC
CGGGGGCTTTCACATCAGACTTAAGAAACCGCCTGCGAGCCCTTTACGCCCA
ATAATTCCGGACAACGCTTGCCACCTACGTATTACCGCGGCTGCTGGCACGTA
GTTAGCCGTGGCTTTCTGGTTAGGTACCGTCAAGGTACCGCCCTATTCGAACG
GTACTTGTTCTTCCCTAACAACAGAGCTTTACGATCCGAAAACCTTCATCACT
CACGCGGCGTTGCTCCGTCAGACTTTCGTCCATTGCGGAAGATTCCCTACTGC
TGCCTCCCGTAGGAGTCTGGGCCGTGTCTCAGTCCCAGTGTGGCCGATCACC

CTCTCAGGTCGGCTACGCATCGTTGCCTTGGTGAGCCGTTACCTCACCAACTA
GCTAATGCGCCGCGGGTCCATCTGTAAGTGGTAGCCGAAGCCACCTTTTATGT
TTGAACCATGCGGTTCAAACAACCATCCGGTATTAGCCCCGGTTTCCCGGAG
TTATCCCAGTCTTACAGGCAGGTTACCCACGTGTTACTCACCCGTCCGCCGCT
AACATCAGGGAGCAAGCTCCCATCTGTCCGCTCGACTTGCATGTATTAGGC

Therefore, the strain of the present invention was finally determined as *Bacillus subtilis* and named as *Bacillus subtilis* Diao 1502.

The fibrinolysin produced by the strain of *Bacillus subtilis,* nattokinase, provided by the present invention has the feature of HPLC spectrogram as follows:
HPLC Detection Conditions:
   Chromatographic column: Waters Protein-Pak™ 60 7.8×300 mm
   Mobile phase: 0.2 mol/L NaH₂PO₄-CH₃OH (95:5)
   Flow rate: 1 ml/min
   Detection wavelength: 220 nm
   The peak concentration of nattokinase production appears between 7 and 9 minutes.

The fibrinolysin produced by the strain of *Bacillus subtilis,* nattokinase, provided by the present invention has the feature of SDS-PAGE electrophorogram as follows:

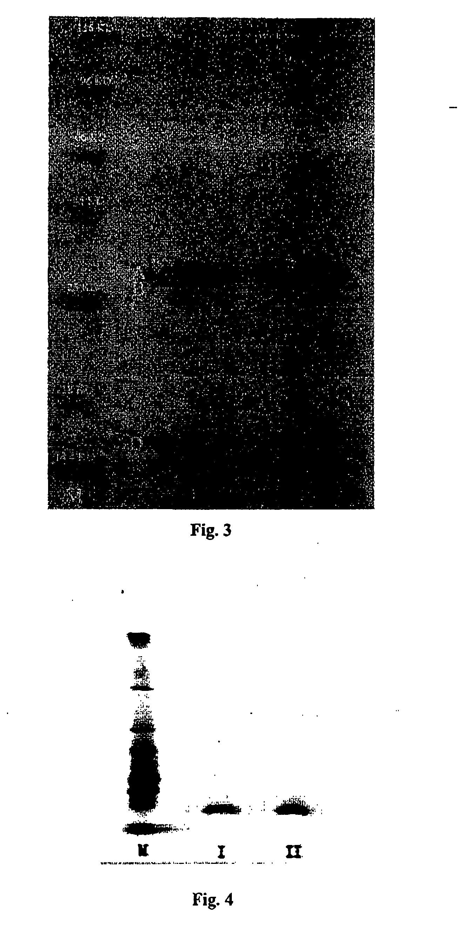

According to the method specified in appendix IV C, Part HI, Edition 2005 of Pharmacopoeia of the People's Republic of China, the nattokniase showed multiple protein bands on SDS-PAGE between 28 KD to 14.4 KD, in which, A, B, C and D bands are clear-cut. See I of Fig. 3 for details.

The fibrinolysin produced by the strain of *Bacillus subrilis,* nattokinase, provided by the present invention has isoelectric focusing electrophoresis electrophorogram feature as follows:

Isoelectric focusing electrophoresis of nattokniase shall be performed according to the method specified in appendix IV D, Part EI, Edition 2005 of Pharmacopoeia of the People's Republic of China. See I of Fig. 4. for details.

The nattokinase of the present invention is prepared by the following method:
a) culturing the *Bacillus subtilis* Diao 1502 (CCTCC NO.M204092) as the strain seed liquid for fermentation;
b) fermenting the strain seed liquid obtained in step a) by liquid state fermentation or solid state fermentation to obtain the nattokinase.

Wherein, the procedure of liquid state fermentation is as follows:

The strain seed liquid was inoculated into fermentation medium according to 5% to 20% (v/v, the volume of medium is taken as the benchmark) and cultured in shaking flasks for 24 to 72 hours at 30 to 40 degree C, rate of rotation of 180 to 220 rpm, and after centrifugalization, the nattokinase is isolated from supernate by sequential use of filter membrane and drying.

The liquid state fermentation medium comprises of carbon source 5 to 35 g/L, nitrogen source 5 to 45 g/L, Na₂HPO_{4·}12H₂O 0.5 to 3 g/L, NaH₂PO₄·2H₂O 0.5 to 4 g/L, CaC1₂ 0.05 to 1 g/L and MgSO₄·7H₂O 0.05 to 1.5 g/L and shall be sterilized at pH 7.5±1.0 and 121 degrees C for 20 minutes.

Wherein, carbon source (Sucrose) could be replaced by glucose, glycerol, soluble amylum and molasses, and nitrogen source (soya bean peptone) could be replaced by corn steep liquor, yeast extract, beef extract, peptone (vegetable protein or animal protein) and bean milk.

One or several adjuvants such as magnesium stearate, β-schardinger dextrin, gelatine, soybean protein isolate and dextrin should be added by 20% to 40% (W/V) in the aforementioned spray drying procedure.

Aforementioned solid state fermentation:

The bean dregs nutritive medium (weight ration: 20 to 120 ml/1000 g) were boiled at 100 degree C to obtain the nutritive medium, then the 5% to 20% (V/W) of inoculation volume of strain seed liquid pre-prepared was inoculated into the cooled fermentation medium and cultured at 30 to 45 degree C and humidity ≧80% for 12 to 56 hours. Finally, the nattokinase production was obtained after drying.

Wherein, the solid state fermentation medium comprises glycerol 50 to 300 g/L, peptone 50 to 300 g/L, beef extract 50 to 100 g/L, Na₂HPO₄·12H₂O 5 to 20 g/L, NaH₂PO₄·2H₂O 5 to 20 g/L, CaCl₂ 0. 5 to 10 g/L, MgSO₄·7H₂O 0.5 to 10 g/L, at pH 7.5±1.0.

The present invention also provides the preparative method for nattokinase.

### a. Preparative method for the strain seed liquid

The strain of the present invention was isolated from fermented food with decomposing fibrin action. Identified and determined by CCTCC as *Bacillus subtilis* Diao 1502, the strain was deposited with CCTCC (accession number of CCTCC M204092).

Opened, the strain of working seed lots will be cultivated in some nutrient agar (NA) medium plates and then maintained on the incline plane of NA sample tube. Seed cells would be judged as qualified for production after their shape, purity and production capacity arc checked out, (i.e. Take a few culture smear from the incline plane of NA test tube and observe under microscope alter gram staining. All strain seeds that were morphologically uniform and without other microorganisms pollution could be selected as certified seed. The purpose of Production Capacity Examination is to check the production capacity of the strain by fermentation at laboratory level, i.e. the yield of enzyme activity of broth medium per milliliter. All of the strain seeds with production capacity exceeding 1000 IU/ml were selected as certified seeds.) Then, they were put into NB medium for shaking cultivation at 30 to 40 degree C and a rotation rate from 180 to 220 rmp for 8 to 24 hours to obtain the first class seeds, and then, they were inoculated in NB medium (nutrient broth) by 5% to 10% of inoculation amount for shaking cultivation at 30 to 40 degree C and 184 to 220 rpm for 8 to 24 hours to obtain the second class seeds or strain seed liquid used for fermentation.

### b. Fermentation

The fermentation described could be liquid or solid state fermentation.

The culture medium described in the present invention comprises of nutritive medium of nutrient broth (NB) for shake cultivation, nutritive medium nutrient agar (NA) for preparation of flat plate and incline plane, nutritive medium of nutrient broth (NB) containing 0.5% agar for preservation of strain in short period.

Wherein, nutritive medium of NB is composed of peptone 10 g/L, beef extract 5 g/L and sodium chloride 5 g/L, which will be sterilized at 121 degree C and pH 7.1±0.2 for 20 minutes.

Wherein, nutritive medium of NA is composed of peptone 10 g/L, beef extract 5 g/L, sodium chloride 5 g/L and agar 13 g/L, which will be sterilized at 121 degree C and pH 7.14±0.1 for 20 minutes

### Liquid State Fermentation

The liquid state fermentation medium comprises: carbon source (sucrose) 5 to 35 g/L, nitrogen source (soy peptone) 5 to 45 g/L, Na₂HPO₄·12H₂O 0.5 to 3 g/L, NaH₂PO₄·2H₂O 0.5 to 4 g/L, CaCl₂ 0.05 to 1 g/L, MgSO₄·7H₂O 0.05 to 1.5 g/L, which will be sterilized at 121 degree C at pH 7.5±1.0 for 20 minutes.

Wherein, carbon source could be replaced by glucose, glycerol, soluble amylum and molasses, while nitrogen source could be replaced by corn steep liquor, yeast extract, beef extract, peptone (vegetable protein or animal protein) and bean milk.

The strain seed liquid prepared was inoculated into fermentation medium with inoculation amount of 5% to 20% and cultured in shaking flasks at the temperature from 30 to 40 degree C and at a rotation rate from 180 to 220 rpm for 24 to 72 hours, and then the nattokinase with the thrombolytic activity was isolated from the supernate by sequential use of concentration with membranes of the molecule weight cut-off of 8000 Da, addition of adjuvants and drying spray.

Addition of adjuvants is principally to meet the needs of drying spray and the adjuvants includes one or more kinds of magnesium stearate, β-schardinger dextrin, gelatine, soybean protein isolate and dextrin in amount of 20% to 40% (W/V).

### Solid State Fermentation

The nutrient fluid of solid state fermentation medium comprises: glycerol 50 to 300 g/L, peptone 50 to 300 g/L, beef extract 50 to 100 g/L, Na₂HPO₄·12H₂O 5 to 20 g/L, NaH₂PO₄·2H₂O 5 to 20 g/L, CaCl₂ 0. 5 to 10 g/L and MgSO₄·7H₂O 0.5 to 10 g/L at pH 7.5±1.0.

The fresh bean dregs were sprayed with nutrient fluid (weight ratio: 20 to 120 ml/1000 g) and boiled at 100 degree C to obtain the nutritive medium, and then the prepared strain seed liquid was inoculated into the cooled fermentation medium mixed with nutrient fluid at the inoculation amount of 5% to 20% and cultured at 30 to 45 degree C with a humidity of ≧ 80% for 12 to 56 hours. Finally, the nattokinase of this invention was obtained after drying. It is important to maintain the humidity and temperature by airiness during fermentation process because the temperature, humidity and nutrient fluid in fermentation process are all key factors to affect the yield of nattokinase.

The nattokinase (NK) of the present invention is a fibrinolysin produced by a strain of *Bacillus subtilis,* which is isolated and purified from food natto that is rich in the same. Existing experiments showed that the activity of the nattokinase is stable at pH values from 6.0 to 12.0 and unstable when pH value is lower than 5.0, and its activity keeps basically unchanged if incubated at the temperature below 56 degree C for 30 minutes. Its pI is 8.6±0.3 by isoelectric focusing electrophoresis. However, the curative effect of oral administration is low because the activity of nattokinase is unstable at low intragastric pH value. Fujita M *et al* reported that after intraduodenal administration in rats, nattokinase (NK) and its complex with the resolvent of fibrinogen could be detected in plasma, suggesting NK could still play the action of thrombolysis after it is absorbed into blood from intestinal tract. (Fujita M, Hong K, Ito Y, et al. Transport of nattokinase across the rat intestinal tract. Biol. Pharm. Bull. 1995, 18(9):1194-6.). The NK of the present invention prepared with 5% NaHCO₃ is used for production of drugs for thrombosis that have significant curative effect in oral administration after the instability of NK at low intragastric pH value is overcome. Therefore, the present invention provides a new choice of drugs for thrombosis in clinical practice and of course, it could be given by injection and in other administrative ways because it has low molecular weight.

The acute toxicity test by intragastric administration of NK in mice shows that LD₅₀ is higher than 6.0 g/kg and the carotid artery thrombosis test in rats indicated that NK prepared by 5% NaHCO₃ has apparent antithrombotic effects, displaying certain dose-effect relationship with a wide range of effective dose from 4 to 100 mg/kg. Simultaneously, the single strain of *Bacillus subtilis* also shows certain prolongation of carotid artery thrombosis time in rats.

The new strain of *Bacillus subtilis* of the invention has a higher rate of enzyme production and the peak value for enzyme production could be obtained the next day with NK activity being 1600 IU/ml in fermentation broth. It could be still increased to 1697 IU/ml after optimization of fermentation condition. The NK activity of crude product is 5000-10000 IU/g after drying in solid state fermentation of 10-kg class and it could be greatly improved after expansion of the scale of production. Therefore, the preparation of nattokinase using the present new strain of *Bacillus subtilis* is applicable for industrialized production with shorter fermentation time, convenient procedures, low cost and high productivity.

### DESCRIPTION OF DRAWINGS

FIG. 1 HPLC spectrogram of nattokinase (NK) prepared in EXAMPLE 2, wherein, abscissa indicates the retention time and ordinate represents the absorption value.
FIG. 2 HPLC spectrogram of nattokinase (NK) prepared in EXAMPLE 3, wherein, abscissa indicates the retention time and ordinate presents the absorption value.
FIG. 3 SDS-PAGE electrophorogram of nattokinase (NK) prepared in EXAMPLE 2 (1) and EXAMPLE 3 (II), wherein, M represents Marker, I is the NK example purified by liquid state fermentation and II is the NK example purified by solid state fermentation.
FIG. 4 Isoelectric focusing electrophoresis electrophorogram of nattokinase (NK) prepared in EXAMPLE 2 (I) and EXAMPLE 3 (II), wherein, M represents Marker, I is the NK example purified by liquid state fermentation and II is the NK example purified by solid state fermentation.

The following is the specific implementation examples to prove further the beneficial effects of the present invention. However, they shall not be taken as the limitation to the present invention. All revision, replacement and alternation that come within the technical meaning and range of equivalency of the present invention are intended to be embraced therein.

### DETAILED DESCRIPTION OF THE INVENTION

### Example 1 Preparation of seed liquid

The opened strain of working seed lots was cultivated on two to three pieces of nutrient agar (NA) medium plates and then 8 to 10 typical colonies were selected to preserve on the inclined planes of NA sample tubes after being cultivated respectively. The seeds would be used in production after being examined as qualified in shape, purity and production capacity.

A ring of culture material was taken from the inclined plane of qualified strains and placed into NB medium of 20 ml for shaking culture at 37 degrees C and at the rotation rate of 200 rpm for 20 hours to obtain the first class seeds, and then, it was inoculated in 400 ml NB medium with an inoculation amount of 5% and cultured in shaking flasks at 37 degrees C and at a rotation rate from 180 to 220 rpm for 18 hours to obtain the seed liquid of fermentation.

### Example 2 Preparation of Nattokinase by Method of Liquid State Fermentation

The culture medium of fermentation was composed of sucrose 5 g/L, soy peptone 12 g/L, Na₂HPO₄·12H₂O 0.50 g/L, NaH₂PO₄·2H₂O 2 g/L, CaCl₂ 0.08 g/L and MgSO₄·7H₂O 1.0 g/L, which should be sterilized at 121 degree C and pH 7.5 for 20 minutes. Wherein, sucrose could be replaced by glucose, glycerol, soluble amylum and molasses, and soya bean peptone by corn steep liquor, yeast extract, beef extract, peptone (vegetable protein or animal protein) and bean milk, etc.

250 ml of the strain seed liquid prepared was inoculated into the fermentation medium with a inoculation volume of 12% and shaking cultured at 37 degree C and a rotation rate of 180 rpm for 50 hours, and then centrifuged at 4000 rpm for 30 minutes to remove thallus. The supernate was then concentrated to 10% of the original volume by filter membrane of the molecule weight cut-off of 8000 Da. Then, adjuvants such as magnesium stearate and gelatin were added to make solid content in this solution reach 20%. Finally, the product of plasminokinase of the present invention was obtained after spray drying at 140 degrees C.

### Purification of Nattokinase

The broth was centrifuged at 5000 rpm for 20 minutes. The supernate was collected and then added with ammonium sulphate [(NH₄)₂SO₄] before being loaded into the hydrophobic interaction column of Phenyl Sepharose™ 6 Fast Flow that had been balanced with 20 mM PB-1.5 M (NH₄)_{z}SO₄ at pH 7.0. Finally it was cluted with 750 mM PB for removing hybridprotein and eluted again with 20 mM PB to get the eluent and then the refined product was harvested after freeze drying.

### HPLC Examination

The aforementioned eluant before freeze drying or refined product (prepared into 1 mg/ml solution with 20 mM PB) is used for HPLC detection.

### HPLC Detection Conditions

Chromatography: Waters 600 + 996, chromatographic column: Waters Protein-Pak™ 60 7.8 × 300 mm, mobile phase: 0.2 mol/L NaH₂PO₄-CH₃OH (95:5), flow rate: 1 ml/min, detection wavelength: 220 nm.

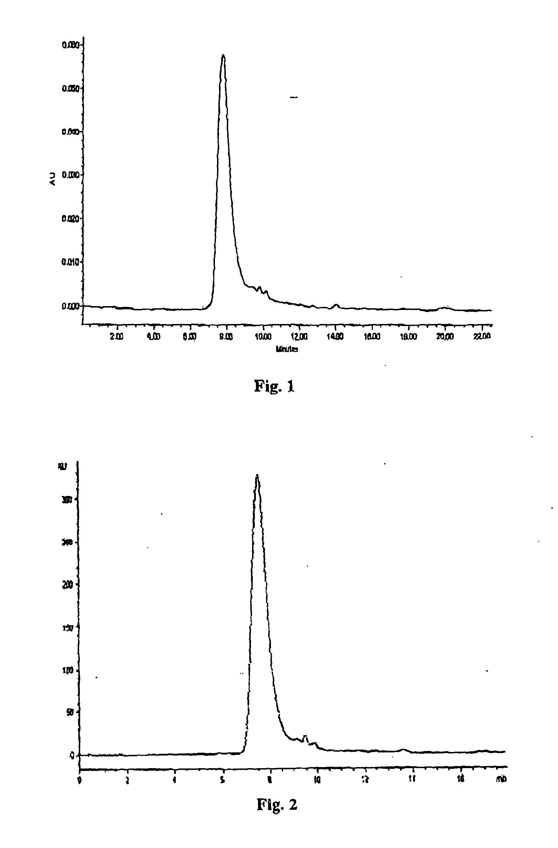

The peak of nattokinase production appears between 7 and 9 minutes. See Fig. 1 for details.

### SDS-PAGE Examination

According to the method specified in appendix IV C, Part III, Edition 2005 of Pharmacopoeia of the People's Republic of China, the nattokinase shall show multiple protein bands on SDS-PAGE between 28 KD and 14.4 KD, in which A, B, C and D bands are most clear-cut. See I of Fig. 3 for details.

### Isoelectric Focusing Electrophoresis Examination

Isoelectric focusing electrophoresis examination shall be performed according to the method specified in appendix IV D, Part III, Edition 2005 of Pharmacopoeia of the People's Republic of China. See I of Fig. 4 for details.

The activity of nattokinase in broth could reach at 1600 IU/ml by detection.

The production of nattokinase would be obtained by drying of the broth or collected eluent.

### Example 3. Preparation of Nattokinase by Method of Solid State

### Fermentation

Nutrient fluid (g/L): glycerol 100 g/L, peptone 150 g/L, beef extract 80 g/L, Na₂HPO₄·12H₂O 18 g/L, NaH₂PO₄·2H₂O 15 g/L, CaCl₂ 10 g/L and MgSO₄·7H₂O 5 g/L, at pH 7.5±1.0.

1000 g fresh bean dregs were sprayed with 100 ml nutrient fluid and then boiled at 100 degree C for 30 minutes. 20% (V/W) of inoculation volume of strain seed liquid pre-prepared was inoculated into the cooled fermentation medium and cultured at 40 degree C with humidity more than or equivalent to 80% for 40 hours. Finally, the nattokinase product was obtained after drying.

### Purification of Nattokinase

Finished product of nattokinase was lixiviated with normal saline of 10 times in volume and treated with ultrasound for 20 minutes and filtrated to gain the supernate, which was afterward prepared into the solution with terminal concentrations of 20 mM PB (pH8.0). After the column of DEAE Sepharose™ Fast Flow was balanced with PB buffer solution of 20 mM PB (pH8.0), it was to be loaded: The solution penetrated through DEAE Sepharose™ Fast Flow column was collected with its pH value adjusted to 6.5, and was then loaded to the CM Sepharose™ Fast Flow column balanced with PB buffer solution of 20 mM PB (pH8.0). The solution eluted with 200 mM PB buffer solution was then collected and added with solid ammonium sulfate [(NH₄)₂SO₄] till the solution concentration turns to 1.5mol/L. Then it was loaded to the hydrophobic interaction column of Phenyl Sepharose™ 6 Fast Flow balanced with buffer solution of 20 mM PB-1.5 M (NH₄)₂SO₄ at pH 7.0 and eluted with 750 mM PB for removal of hybridprotein, and eluted again with 20 mM PB. Finally, the eluent was collected and the refined nattokinase was obtained after freeze drying.

### HPLC Examination

The aforementioned eluant before freeze drying or refined product was prepared into solution of 1 mg/ml with 20 mM PB for HPLC detection.

### HPLC Detection Conditions:

Chromatography: Agilent 1100 series, chromatographic column: Waters Protein-Pak™ 60 7.8×300 mm, mobile phase: 0.2 mol/L NaH₂P0₄-CH₃0H (95:5), flow rate: 1 ml/min, detection wavelength: 220 nm.

The peak of nattokinase production appears between 7 and 9minutes. See Fig. 2 for details.

### SDS-PAGE Examination

According to the method specified in appendix IV C, Part III, Edition 2005 of Pharmacopoeia of the People's Republic of China, the nattokinase shows multiple protein bands on SDS-PAGE between 28 KD and 14.4 KD, wherein, A, B, C and D bands are most clear-cut. See II of Fig. 3 for details.

### Isoelectric Focusing Electrophoresis Examination

Isoelectric focusing electrophoresis examination is performed according to the method specified in appendix IV D, Part III, Edition 2005 of Pharmacopoeia of the People's Republic of China. See the II of Fig. 4 for details.

The final nattokinase product of the present invention is obtained after drying the solid state fermentation medium (after fermentation) or the collected eluent (purification solution).

Multiple batches of the solid state fermentation experiment of 10-Kg class suggested that the rough yield of enzyme activity could be up to 5000-10000 IU/g after torrefaction.

The following is the research of freeze-dried powder of nattokinase on carotid artery thrombosis in rats to further demonstrate the benefits of the present invention.

### Testing Medicine

### Freeze-dried Powder of Nattokinase:

Crude drug, luteous powder with water-solubility and batch No. 20040617, provided by Chen Jun in the microorganism group of Chengdu Di'ao Pharmaceutical Group Company Limited.

Method for Preparation: The mother liquid was formulated by 20 mg/ml with 5% NaHCO₃ and then diluted to solution of 0.8 mg/ml with 5% NaHCO₃ according to geometric proportion for intragastric administration in rats.

Dosage: 4, 20 and 100 mg/kg were set up for low, middle and high doses, respectively.

Basis for Dosage Setting: The maximal dosage for clinical patients is designed as 3.0g each day, i.e. 3000 mg/60 kg=50 mg/kg, which is equivalent to 9.26 mg/kg of the equivalent dose in rats. The integer number twice over it (20 mg/kg) was used as the middle dosage for pharmacodynamic effect in rats. The intragroup times was designed as 5 to expand the range of high, middle and low dosage in order to explore conveniently the suitable scope of therapeutic dosage.

Basis of Preparation: It has been reported that the nattokinase-like drugs are stable at pH values from 6.0 to 12.0 and unstable when to the PH value is below 5.0. Generally, the pH values of digestive tract are ranged from 0.9 to 1-5 in stomach, 6.0 to 6.8 in small intestine and 6.5 to 7.5 in colon. Therefore, the direct intragastric administration of drug containing nattokinase prepared with common solution adjuvant is unstable and easy to breakdown. The preliminary test for the nattokinase prepared by 5% NaHCO₃ suggested that it has manifested no decrease in activity after being placed at room temperature for 4 days.

### Enteric Coated Tablets of Aspirin:

The enteric coated aspirin tablets, produced by Shijiazhuang Pharmaceutical Group OUYI Pharma Co., Ltd of China (batch number:040401), are prepared into 8 mg/ml drug solution with distilled water for intragastric administration in rats.

Basis of Dosage: 80 to 300 mg/kg/day, calculated as 1.33 to 5mg/kg/day for an adult of 60 kg in weight, which would be 8 to 30mg/kg/day after conversion to the dosage for rats. The dosage of aspirin for rats in this experiment was designed to be 40 mg/kglday according to aforementioned dosage and existing test experiences.

### Staphylokinase

Freeze-dried powder of white color, 5 mg/ampoule, batch No. 20040301, water-solubility, provided by Chengdu Di'ao Pharmaceutical Group Company Limited. An ampoule of freeze-dried powder was prepared into 0.3 mg/ml drug solution with distilled water for intravenous injection in rats.

Basis of Dosage: Generally, 15 mg is for a single dose for an adult, i.e. 15 mg/60 kg=0.25 mg/kg. After conversion to the equivalent dosage for rats, it should be ranged from 1.35 to 1.5 mg/kg, and 1.5 mg/kg was taken as the dosage in this test.

Experimental Animal: 84 male SD rats weighing 250 to 320 g, purchased from the Animal Center of Henan Medical University of China.

Experimental Apparatus: Experimental Intracorporeal Thrombosis Surveyor BT87-3, developed by the cardiovascular research department of Baotou Medical Unversity of China.

### Experiment Method:

Eighty-four male SD rats were randomly assigned to seven experimental groups (12 rats each group) according to their body weight. The freeze-dried powder of nattokinase and positive drug aspirin were given to test groups and the control group was administered with 5% NaHCO₃, by intragastric administration according to the dosage of 0.5 ml/100 g each day for 7 continuous days. The positive drug staphylokinase was given at the 7th day by intravenous injection.

The carotid artery of test rats were separated 1 hour after the last intragastric administration or 15 minutes after intravenous injection according to the method prescribed in existing literature. Being stimulated continually with direct current (2 mA) for 7 minutes, the injuries of endarterium of carotid artery formed thrombosis gradually in test rats. The experimental apparatus would give an automatic alarm and record the time of thrombus formation when the blood vessels were blocked.

### Results of the Experiment

1. Changes of weight of test rats in all groups after 7 days of administration were shown as follows (See Table 1 for details).

**Table 1**

| No | Groups | Animal No. | Before administration | After 7 days of administration |
|---|---|---|---|---|
| A | Negative control | 12 | 293±20 | 307±21 |
| B | Positive control (Aspirin) | 12 | 296±15 | 316±20 |
| C | Positive control (staphylokinase) | 12 | 293±22 | 316±21 |
| D | Low dosage of nattokinase of the invention | 12 | 298±18 | 315±20 |
| E | Middle dosage of nattokinase of the invention | 12 | 296± 19 | 316±21 |
| F | High dosage of nattokinase of the invention | 12 | 291±23 | 311±23 |
| G | Strains of *Bacillus subtilis* | 12 | 295±16 | 328±20 |

The results showed that the weight of test rats had no significant difference between groups before administration and, after 7 days of administration, the weight of test rats in all groups increased steadily and with no significant difference. The rats in each group all had normal eating, drinking and action during administration period and showed no other overt toxicity either. In conclusion, the nattokinase drug prepared with 5% NaHCO₃ has no apparent toxicity when it is given to rats during the period of 7-day's intragastric administration.
2. Thrombus formation time (second) is compared as follows (See the Table. 2 for details):

**Table 2**

| **Groups** | **Negative control** | **Aspirin** | **Staphylokinase** | **Low dosage of nattokinase of the invention** | **Middle dosage of nattokinase of the invention** | **High dosage of Nattokinase of the invention** | **Strains of *Bacillus subtilis*** |
|---|---|---|---|---|---|---|---|
| Case No. | 12 | 8 | 10 | 11 | 11 | 10 | 9 |
| Mean value | 943 | 1379** | 1224* | 1237* | 1287* | 1309** | 1198* |
| Certified value | 164 | 285 | 196 | 275 | 327 | 219 | 234 |
| P value | | 0.0049 | 0.0132 | 0.0446 | 0.0181 | 0.006 | 0.0289 |

Results of the experiment shows that the administration of freeze dried powder of nattokinase in all groups of low, middle and high dosage compared with negative control group prolonged significantly the carotid artery thrombosis time in rats (*P*<0.05 or *P*<0.01). There appeared certain dose-effect tendency between different dosage groups in spite of the absence of significant dose-effect relationship. Compared with positive Aspirin, groups of different dosages showed no significant difference.

In addition, the single strain of *Bacillus subtilis* also showed certain prolongation in carotid artery thrombosis time in rats (*P*<0.05).

On the whole, the strain itself or the nattokinase produced by it in the present invention provides a new selection of drugs for treating thrombosis.

### INDUSTRIAL APPLICABILITY

The new strain of *Bacillus subtilis* of the invention has a higher rate of enzyme production and the peak value for enzyme production could be obtained the second day. Therefore, producing nattokinase using the present new strain of *Bacillus subtilis* is of shorter fermentation time, convenient procedures, low cost and high productivity, thus providing a new way for the industrialized production of nattokinase drugs or preparations.

## Claims

1. A use of a new strain of the *Bacillus subtilis* Diao 1502 which is deposited in China Center for Type Culture Collection with an accession number of CCTCC M204092 in preparing pharmaceuticals for treating thrombosis.

2. The use of claim 1, wherein the use of nattokinase produced by *Bacillus subtilis* Diao 1502 with an accession number of CCTCC M204092 in preparing pharmaceuticals for treating thrombosis.

3. The use of claim 2, wherein the HPLC spectrogram of the said nattokinase has a characteristic absorption peak displayed in Fig. 1, the chromatographic condition is as follows:
chromatographic column: Waters Protein-Pak™ 60 7.8×300 mm
mobile phase: 0.2 mol/L NaH₂PO₄-CH₃OH (95:5)
flow rate: 1 ml/min
detection wavelength: 220 nm
the peak concentration of nattokinase appears between 7 and 9 minutes.

4. A kind of nattokinase having a HPLC spectrogram displayed in Fig. 1, wherein said HPLC spectrogram has one characteristic absorption peak, the chromatographic condition is as follows:
chromatographic column: Waters Protein-Pak™ 60 7.8×300 mm
mobile phase: 0.2 mol/L NaH₂P0₄-CH₃0H (95:5)
flow rate: 1 ml/min
detection wavelength: 220 nm
the peak concentration of nattokinase appears between 7 and 9 minutes.

5. The nattokinase of claim 4, wherein said nattokniase shows multiple protein bands on SDS-PAGE between 28 KD and 14.4 KD.

6. The nattokinase of claim 4 or claim 5, wherein said nattokinase is prepared by the following method:
a). culturing the *Bacillus subtilis* Diao 1502 (CCTCC NO.M204092) as the strain seed liquid for fermentation;
b). fermenting the strain seed liquid obtained in step a) by liquid state fermentation or solid state fermentation to obtain the nattokinase.

7. A method of preparing the nattokinase of claim 4 or 5, comprising the steps of:
a). culturing the *Bacillus subtilis* Diao 1502 (CCTCC NO.M204092) as the strain seed liquid for fermentation;
b). fermenting the strain seed liquid obtained in step a) by liquid state fermentation or solid state fermentation to obtain the nattokinase.

8. The method of claim 7, wherein said liquid state fermentation comprises the steps of:
inoculating the strain seed liquid into fermentation medium by 5% to 20% (v/v);
culturing said strain seed liquid in shaking flasks for 24 to 72 hours at 30 to 40 degree C, rate of rotation being 180 to 220 rpm:
centrifugalizing and concentrating the supernate obtained in culture through filter membrane; and
drying of which to obtain the nattokinase.

9. The method of claim 8, wherein the medium of said liquid state fermentation comprises: carbon source 5 to 35 g/L, nitrogen source 5 to 45 g/L, Na₂HPO₄·12H₂O 0.5 to 3 g/L, NaH₂PO₄·2H₂O 0.5 to 4 g/L, CaCl₂ 0.05 to 1 g/L, MgSO₄·7H₂O 0.05 to 1.5 g/L, at pH 7.5±1.0; wherein said carbon source is selected from a group consisting of sucrose, glucose, glycerol, soluble amylum and molasses, and said nitrogen source is selected from a group consisting of animal peptone, corn steep liquor, yeast extract, beef extract, vegetable peptone and bean milk.

10. The method of claim 7, wherein said solid state fermentation comprises the steps of:
boiling the bean dregs nutritive medium in weight ratio 20 to 120 ml/1000 g at 100 degree C to obtain the nutritive medium;
inoculating the strain seed liquid in 5% to 20% (V/W) of inoculation volume into the cooled nutritive medium and culturing at 30 to 45 degree C and humidity ≧ 80% for 12 to 56 hours;
drying the culture to obtain the nattokinase.

11. The method of claim 10, wherein the medium of said solid state fermentation comprises: glycerol 50 to 300 g/L, peptone 50 to 300 g/L, beef extract 50 to 100 g/L, Na₂HPO₄·12H₂O 5 to 20 g/L, NaH₂PO₄·2H₂O 5 to 20 g/L, CaCl₂ 0.5 to 10 g/L, MgSO₄·7H₂O 0.5 to 10 g/L. at pH 7.5±1.0.
